# DEMANDE DE BREVET EUROPEEN

(11) **EP 3 797 769 A1**
(43) Date de publication de la demande: **31.03.2021**
(21) Numéro de dépôt: 19315118.0
(22) Date de dépôt: 25.09.2019
(51) Int. Cl.: A61K 31/40, A61P 25/02

(54) **COMPOSITION A USAGE THERAPEUTIQUE**

(71) Demandeur: Fontès, M. Michel, 20214 Calenzana (FR)
(72) Inventeur: Fontès, Michel, 20214 Calenzana (FR)
(74) Mandataire: Kessler, Marc

(57) **Abrégé**

La présente invention se rapporte à un composé de formule (I), et une composition comprenant un tel composé, 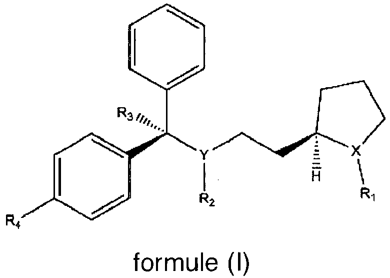 dans laquelle X et Y, sont indépendamment l'un de l'autre choisis parmi N, O, ou S, R₁ est choisis parmi l'hydrogène, un alkyl linéaire ou branché, de préférence CH₃, si X est N, R₂ est choisis parmi l'hydrogène, un alkyl linéaire ou branché, si Y est N, R₃ est choisi parmi l'hydrogène, un alkyl linéaire ou branché, de préférence CH₃, R₄ est choisi parmi -Cl, -CF₃, -CH₃, destiné a être utilisé dans le traitement de la maladie de Charcot-Marie-Tooth liée au chromosome X (CMTX), et des troubles associés.

## Description

### Objet de l'invention

La présente invention se rapporte à une composition pour la prévention ou le traitement de maladies du système nerveux périphérique, ou des troubles associés à ces maladies, et en particulier le traitement ou la prévention de la maladie Charcot-Marie-Tooth liée au chromosome X (CMTX), et encore plus particulièrement du sous-type 1, ou CMTX1.

### Etat de la technique

Les troubles neurologiques sont des maladies chroniques du système nerveux central ou du système nerveux périphérique, invalidantes et bien souvent à évolution lente. Même si leur diagnostique est de nos jours plus aisé, leur traitement consiste bien souvent en la réduction des symptômes associés à ces maladies, sans véritablement pouvoir empêcher leur apparition ou leur progression.

Parmi les maladies touchant le système nerveux central, la maladie d'Alzheimer et la Sclérose En Plaque (SEP) ont été largement étudiées, et de nombreuses familles de molécules ont été proposées pour leur traitement.

Par exemple, le document WO2004000831 propose l'emploi d'antagonistes des récepteurs de l'histamine H3, de la famille des indoles, pour le traitement de la maladie d'Alzheimer, ou bien, comme cela est décrit dans le document US6100279, des antagonistes des antagonistes des récepteurs de l'histamine H3 de la famille des imidazoles, en combinaison avec des antagonistes du récepteur d'histamine H1, comme la clémastine, une pyrrolidine, qui est connue pour traiter les maladies ou troubles liés à une libération d'histamine, comme par exemple les éternuements, les écoulement nasal, le larmoiement, les démangeaisons et autres symptômes du rhume ou des allergies.

Le document WO2018022904 suggère également l'emploi de la clémastine dans le traitement de la Sclérose en Plaque par son action de myélinisation ou rémyélinisation des neurones, grâce à une amélioration ou incitation à l'accumulation d'intermédiaires stérols Δ8,9-insaturés de la voie de la biosynthèse du cholestérol, dans les cellules progénitrices d'oligodendrocytes pouvant induire la génération d'oligodendrocytes.

Parmi les maladies touchant le système nerveux périphérique, la maladie de Charcot-Marie-Tooth (CMT) est la neuropathie périphérique héréditaire la plus fréquente, affectant environ une personne sur deux mille cinq cent. Elle se caractérise par une dégénérescence neuromusculaire progressive, une perte progressive de tissu musculaire et de la sensation tactile à travers diverses parties du corps.

La CMT présente une grande hétérogénéité génétique. Sous une même appellation, on trouve des maladies dues à des gènes différents, situés sur des chromosomes différents. Ainsi, il existe différentes formes de CMT, classés selon la partie du nerf atteinte (myéline ou axone,) le mode de transmission génétique (dominante ou récessive ou lié au chromosome X) et selon l'anomalie génétique causant de la maladie. Ainsi, il existe, à ce jour, six formes de CMT (CMT1, CMT2, CMT4, CMTX, DI-CMT, RI-CMT), chaque forme étant divisée en sous-types en fonction de l'anomalie génétique en cause.

La variante lié au chromosome X (CMTX) se distingue par l'apparition d'une faiblesse musculaire distale, progressive, modérée à sévère, et d'une atrophie des extrémités inférieures et des muscles intrinsèques des mains, associée à des pieds creux, un pied tombant bilatéral, une réduction ou une absence de réflexes tendineux, et une atteinte sensorielle légère à modérée des extrémités inférieures. La CMTX se caractérise par un mode de transmission dominant, lié au chromosome X, et regroupe six sous-types qui sont fonction de la mutation touchant différents gènes du chromosome X. Le sous-type CMTX1 est lié à une mutation du gène GJB1 codant pour la protéine de connexine 32 (Cx32), également appelée protéine beta-1 à jonction lacunaire (GJB1), une protéine transmembranaire impliquée dans la formation de jonctions de communication intercellulaire entre des cellules voisines, chaque jonction étant formée par deux demi-canaux, ou connexons, dont chacun est lui-même constitué de six molécules de connexine. La proximité spatiale de deux connexons dans un espace intercellulaire déclenche la formation d'un canal qui relie les cytoplasmes de cellules adjacentes et permet l'échange d'ions, de petites molécules (<1000 Da) et d'effecteurs de signalisation. Cette fonction connexon est très réduite chez les patients soufrant de la variante CMTX.

Le document WO2014095757 décrit l'utilisation de sulfonamides, la KN93 et la KN62, des inhibiteurs de la protéine kinase Ca²⁺ / calmoduline-dépendante II, ou CaMKII, pour le traitement de la variante CMTX de la maladie de Charcot-Marie-Tooth. Toutefois, la mise au point et le développement d'une composition pharmaceutique, ou d'un médicament, ayant comme principe actif la KN93 ou la KN62 s'avère difficile et couteux de part la nature même des molécules en question. De plus, une telle composition ne peut être administrée par voie orale, mais seulement par voie intraveineuse, ce qui rend son utilisation au quotidien peu aisée.

### Buts de l'invention

La présente invention vise à fournir un composé et une composition pharmaceutique, qui ne présentent pas les inconvénients de l'état de la technique.

La présente invention vise à fournir un traitement alternatif à ceux connus pour traiter la variante CMTX de la maladie Charcot-Marie-Tooth, en particulier le sous-type CMTX1.

### Résumé de l'invention

La présente invention porte sur un composé de formule (I) : 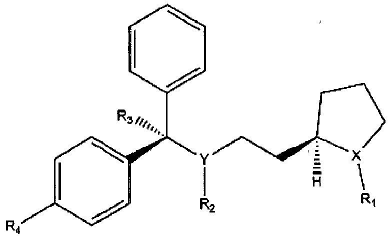 dans laquelle X et Y, sont indépendamment l'un de l'autre choisis parmi N, O, ou S, R₁ est choisis parmi l'hydrogène, un alkyl linéaire ou branché, de préférence CH₃, si X est N, R₂ est choisis parmi l'hydrogène, un alkyl linéaire ou branché, si Y est N, R₃ est choisi parmi l'hydrogène, un alkyl linéaire ou branché, de préférence CH₃, R₄ est choisi parmi -Cl, -CF₃, -CH₃, destiné a être utilisé dans le traitement de la maladie de Charcot-Marie-Tooth liée au chromosome X (CMTX), et des troubles associés.

Selon des modes particuliers de l'invention, le composé selon l'invention est utilisé pour traiter le sous-type 1 de la maladie Charcot-Marie-Tooth (CMTX1), et de préférence, le composé est la clémastine.

La présente invention porte également sur une composition comprenant le composé de formule (I) selon l'invention, et un excipient ou véhicule pharmaceutiquement acceptable, destiné à être utilisé dans le traitement ou la prévention de la maladie de Charcot-Marie-Tooth liée au chromosome X (CMTX) ou d'un trouble associé

Selon des modes particuliers de l'invention, la composition selon l'invention comprend au moins une, ou une combinaison quelconque appropriée, des caractéristiques suivantes :
- la maladie de Charcot-Marie-Tooth liée au chromosome X est du sous-type 1 (CMTX1),
- le composé de formule (I) est la clémastine,
- le composé de formule (I) est associé à un contre ion qui est le diméthylfumarate,
- la composition est utilisée pour une prise orale journalière,
- la composition est utilisée après la détermination que le patient à traiter avec ladite composition est atteint de la variante liée au chromosome X de la maladie Charcot-Marie-Tooth (CMTX) ou que le patient est atteint du sous-type 1 de la variante liée au chromosome X de la maladie Charcot-Marie-Tooth (CMTX1),

La présente invention porte également sur l'utilisation de la clémastine en tant qu'agent activateur de l'activité connexon chez des patients souffrant de la variante liée au chromosome X de la maladie Charcot-Marie-Tooth (CMTX) ou du sous-type 1 de la variante liée au chromosome X de la maladie Charcot-Marie-Tooth (CMTX1).

### Brève description des figures

La figure 1 représente le pourcentage de noyaux de cellules de fibroblastes d'un patient souffrant de CMTX, avec ou sans traitement avec le composé selon l'invention.

La figure 2 représente l'activité connexon, évaluée par internalisation de LY sur des cellules de fibroblastes d'un patient souffrant de CMTX, avec ou sans traitement avec le composé selon l'invention.

### Description détaillée de l'invention

Dans la suite de description de la présente invention, les termes « troubles associés à la CMTX » désigne toutes autres neuropathies périphériques à la fois héréditaires et acquises, qui sont associés à la variante liée au chromosome X ou CMTX, et qui sont liées à des mutations touchant différents gènes du chromosome X. Les termes « traitement » ou « traiter » incluent la thérapie en tant que telle, mais également le retard ou la réduction de la progression de la maladie et des symptômes qui y sont associés, ainsi que de la douleur provoquée par le trouble. Les termes « prévention » et « prévenir » incluent également la prophylaxie de la maladie.

Le composé selon l'invention de formule générale (I) : 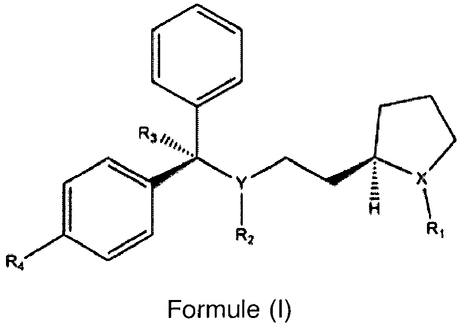
dans laquelle X et Y, sont indépendamment l'un de l'autre choisis parmi N, O, ou S, R₁ est choisis parmi l'hydrogène, un alkyl linéaire ou branché, de préférence CH₃, si X est N, R₂ est choisis parmi l'hydrogène, un alkyl linéaire ou branché, si Y est N, R₃ est choisi parmi l'hydrogène, un alkyl linéaire ou branché, de préférence CH₃, R₄ est choisi parmi -Cl, -CF₃, -CH₃,
ledit composé étant destiné à être utilisée dans le traitement de la maladie de Charcot-Marie-Tooth liée au chromosome X (CMTX), en particulier du sous-type CMTX1, et des troubles associés à cette maladie, chez n'importe quel sujet mammifère, en particulier chez l'homme.

De préférence, le composé de formule (I) est sous la forme d'un sel, en association avec un contre ion, qui de préférence est choisi parmi HCl, le tartrate, le succinate ou le diméthyl fumarate.

De préférence, le composé de formule I est la clémastine, avec X est un atome d'azote (N), Y est un atome d'oxygène (O), R₁ et R₃ sont CH₃ et R₄ est Cl. La clémastine est la (+)-2-[2-[(p-chloro-a-methyl-a-phenylbenzyl)oxy]ethyl]-1-ethylpyrrolidine.

A titre d'exemple, l'activité de la clémastine, pour le traitement ou la prévention de la maladie CMTX, a été étudiée sur des fibroblastes provenant d'un individu ne souffrant pas de CMTX et sur une culture de cellules de fibroblastes humain, obtenue d'un patient soufrant de CMTX. Le patient présente des parésies de la flexion dorsale du pied, de la flexion plantaire, de légères parésies de l'abduction des doigts et une insensibilité du pied, ainsi qu'un pied creux et des doigts de pieds en marteau. Les reflexes des tendons sont absents dans les bras et les pieds. La vitesse de conduction des nerfs est de 34,1 m/s et la mesure de l'amplitude du signal électrique (CAMP) est de 1,4mV.

Un échantillon de 1mm de peau a été prélevé sur le patient est transféré dans un médium de culture DMEM (Dulbecco's Modified Eagle Médium) supplémenté avec 20% de sérum de veau foetal, de la pénicilline, la streptomycine et de la glutamine (milieu D20). L'échantillon est ensuite transféré dans une boite de pétri et recouvert de médium D20. Après que des fibroblastes aient grandis, l'échantillon a été retiré et les cultures ont été cultivées et sont passés trois fois, c'est-à-dire détachées du support pour une resuspension dans du milieu de culture neuf, avant que les essais aient lieu.

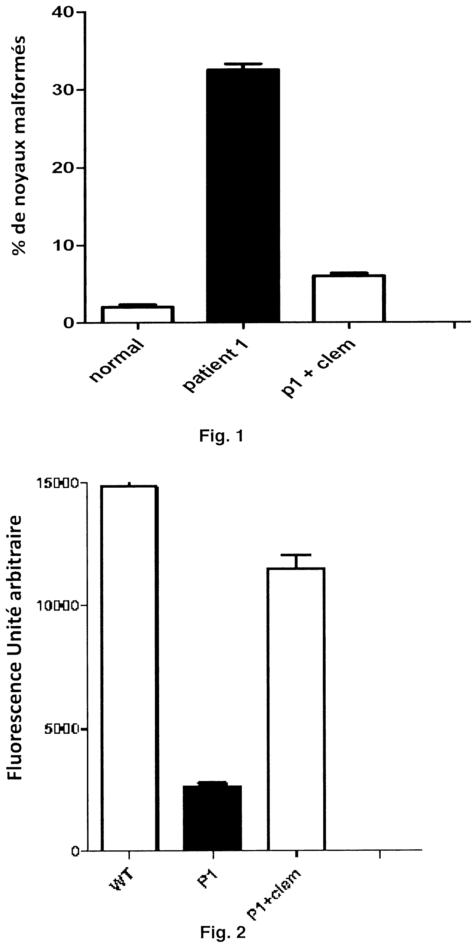

Les anomalies des noyaux cellulaires sont étudiées en déposant les fiboblastes sur des lames de microscopie et en colorant les noyaux à de l'aide 4',6-diamidino-2-phénylindole, ou DAPI, les rendant ainsi fluorescents. Les anomalies nucléaires sont classées selon la classification établie par le Mitocheck, à savoir : i) les noyaux mal formés, ii) les noyaux polylobés, iii) les noyaux non séparés et iv) les autres. Le pourcentage des noyaux anormaux chez le patient atteint de CMTX1 est ensuite quantifié avec ou sans traitement avec de la clémastine, à une dose de 10*µ*mole, et les résultats sont comparés avec ceux obtenus avec des fibroblastes humains provenant d'individus ne présentant pas de CMTX1 (Figure 1).

L'activité connexon (Figure 2) a été étudiée sur une culture de cent mille cellules cultivée pendant un jour avec ou sans clémastine, à une concentration finale de 10 *µ*M, à l'aide du jaune de Lucifer (LY), ajouté au milieu à une concentration finale de 110 *µ*M, et par une incubation de deux heures est effectuée. Le jaune de Lucifer est peu fluorescent à l'extérieur des cellules, mais devient fluorescent dans le cytoplasme. Ainsi, la fluorescence résultante, enregistrée en utilisant un lecteur de microplaque Perkin Elmer Victor 4 (excitation: 405 nM, émission: 535 nM), permet de quantifier la quantité de jaune de Lucifer ayant pénétré dans les cellules via les connexons.

L'analyse statistique des résultats a été faite en utilisant le logiciel Prism V5.0 de Mann-Whitney et des tests Chi carré ont été utilisé pour une analyse de tendance, le niveau de confiance étant fixé à 95%.

Comme le montre la figure 1, les noyaux anormaux, chez le patient souffrant de CMTX1, représentent environ 30% de la totalité des noyaux observés, contre moins d'1% chez un individu ne souffrant pas d'une telle pathologie (Figure 2). Le traitement des cellules du patient à l'aide de clémastine réduit sensiblement le nombre de noyaux anormaux, à environ 5%.

S'agissant de l'activité connexon, et comme le montre la figure 2, il apparaît que la clémastine restaure l'activité connexon dans les cellules du patient souffrant de CMTX, et en particulier de CMTX1.

Par conséquent, la clémastine peut être utilisée en tant qu'activateur de l'activité connexon et l'utilisation de la clémastine apparaît être un traitement efficace pour les patients atteints de CMTX1.

La composition selon l'invention comprend une quantité thérapeutiquement efficace d'un composé de formule I, de préférence de clémastine, ou d'un pro-médicament ou bioprécuseur de celui-ci et au moins un véhicule et/ou excipient pharmaceutiquement acceptable.

Cette quantité thérapeutiquement efficace dépend du mode d'administration de la composition selon l'invention, du poids corporel, de l'âge du patient, de la gravité des dommages neuropathiques causés par la maladie CMTX et/ou le risque d'effets secondaires potentiels compte tenu de l'état de santé général de la personne à traiter. Elle peut être estimée initialement soit dans des dosages de culture cellulaire, soit dans des modèles animaux, habituellement des souris, des rats, des porcs, des lapins, des chiens ou des porcs. Le modèle animal peut également être utilisé pour déterminer la plage de concentration appropriée et la voie d'administration.

De préférence, l'utilisation d'un composé de formule I, de préférence de la clémastine, ou d'une composition pharmaceutique comprend la détermination préalable si le sujet à traiter est atteint de CMTX, et en particulier de la CMTX1, une détermination qui peut être effectué par divers tests connus en soi, tels que des tests d'ADN.

La composition selon l'invention est toute composition adéquate pour une administration orale, rectale, topique (notamment transdermique, buccale et sublinguale) ou parentérale (notamment sous-cutanée, intramusculaire, intraveineuse et intradermique). De préférence, la composition est administrée oralement par une prise par jour.

La composition selon l'invention peut également être formulées pour libérer le composé de formule I, de préférence la clémastine, pratiquement immédiatement après l'administration ou à tout moment prédéterminé après l'administration, ou pour une administration prolongée ou contrôlée.

Selon la voie d'administration choisie, la composition selon l'invention est sous forme solide ou liquide, une forme appropriée à l'administration en question, sous forme de comprimés, de poudre, de solutions ou de suspensions. La composition selon l'invention se trouve de préférence sous une forme unitaire, à savoir une forme contenant une quantité prédéterminée de matière active calculée pour produire l'effet thérapeutique souhaité, en association avec un excipient pharmaceutique approprié, qui peut être un comprimé, pilule, capsule ou analogue, ampoule ou seringue.

Le véhicule et/ou l'excipient pharmaceutiquement acceptable, par exemple, des diluants ou des charges inertes, comme par exemple du saccharose, des cyclodextrines du carbonate de calcium, du chlorure de sodium, du phosphate de calcium, du sulfate de calcium ou du phosphate de sodium, des agents de granulation et de désintégration, des liants, des agents lubrifiants, des agents glissants, des anti-adhésifs, et une combinaison de ceux-ci. D'autres excipients pharmaceutiquement acceptables peuvent être des colorants, des agents aromatisants, des plastifiants, des humectants, des conservateurs, des agents tampons et similaires.

Pour une administration orale, les comprimés peuvent être non enrobés, ou être enrobés par des techniques connues, éventuellement pour retarder la désintégration et l'absorption dans le tractus gastro-intestinal, procurant ainsi une action soutenue pendant plus longtemps, ou une libération selon un motif prédéterminé. Les poudres peuvent être dispersables ou sous forme de granules pour la préparation d'une suspension aqueuse par addition d'eau, l'ingrédient actif étant utilisé en combinaison avec un agent dispersant ou mouillant, un agent de suspension et un ou plusieurs conservateurs.

Pour une administration parentérale, la composition peut être incorporée dans tous dispositifs d'administration ou implants appropriés, contenant des supports classiques, non toxiques, pharmaceutiquement acceptables et adjuvants, sous forme de doses unitaires ou de des flacons contenant plusieurs doses. De préférence, les supports sont biocompatibles et peuvent être par exemple des glucides, des protéines ou des lipoprotéines.

Quelque soit le mode d'administration, la composition selon l'invention peut être incorporée dans des microsphères, des microcapsules, des nanoparticules, des liposomes ou similaires, pour une libération contrôlée.

L'administration de la composition selon l'invention peut se faire de préférence une fois par jour, mais peut également l'être plusieurs fois par jour pendant plusieurs jours à plusieurs années et peut même durer toute la vie du patient.

## Revendications

1. Composé de formule (I) : 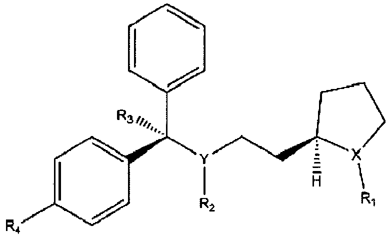 dans laquelle X et Y, sont indépendamment l'un de l'autre choisis parmi N, O, ou S, R₁ est choisis parmi l'hydrogène, un alkyl linéaire ou branché, de préférence CH₃, si X est N, R₂ est choisis parmi l'hydrogène, un alkyl linéaire ou branché, si Y est N, R₃ est choisi parmi l'hydrogène, un alkyl linéaire ou branché, de préférence CH₃, R₄ est choisi parmi -Cl, -CF₃, -CH₃, destiné a être utilisé dans le traitement de la maladie de Charcot-Marie-Tooth liée au chromosome X (CMTX), et des troubles associés.

2. Le composé de formule (I) pour son utilisation selon la revendication 1, dans laquelle la maladie Charcot-Marie-Tooth est du sous-type 1 (CMTX1).

3. Le composé de formule (I) pour son utilisation selon l'une quelconque des revendications 1 ou 2, dans laquelle ledit composé est la clémastine.

4. Une composition comprenant le composé de formule (I), et un excipient ou véhicule pharmaceutiquement acceptable, destiné à être utilisé dans le traitement ou la prévention de la maladie de Charcot-Marie-Tooth liée au chromosome X (CMTX) ou d'un trouble associé.

5. La composition pour son utilisation selon la revendication 4 dans laquelle la maladie Charcot-Marie-Tooth est du sous-type 1 (CMTX1).

6. La composition pour son utilisation selon l'une quelconque des revendications 4 ou 5, dans laquelle le composé de formule (I) est la clémastine.

7. La composition pour son utilisation selon l'une quelconque des revendications 4 à 6, dans laquelle ladite composition comprend le composé de formule I et un contre ion étant le diméthylfumarate.

8. La composition pour son utilisation selon l'une quelconque des revendications 4 à 7, pour une prise orale journalière.

9. La composition pour son utilisation selon l'une quelconque des revendications 4 à 8, dans laquelle l'utilisation de ladite composition comprend la détermination que le patient à traiter avec ladite composition est atteint de la variante liée au chromosome X de la maladie Charcot-Marie-Tooth (CMTX) ou que ledit patient est atteint du sous-type 1 de la variante liée au chromosome X de la maladie Charcot-Marie-Tooth (CMTX1).

10. La clémastine, destinée à une utilisation en tant qu'agent activateur de l'activité connexon chez des patients souffrant de la variante liée au chromosome X de la maladie Charcot-Marie-Tooth (CMTX) ou du sous-type 1 de la variante liée au chromosome X de la maladie Charcot-Marie-Tooth (CMTX1).
